# EUROPEAN PATENT APPLICATION

(11) **EP 1 857 552 A1**
(43) Date of publication of application: **21.11.2007**
(21) Application number: 06010465.0
(22) Date of filing: 20.05.2006
(51) Int. Cl.: C12N 15/61, C12N 9/92, C12P 19/24, A23L 1/09

(54) **Thermostable xylose isomerase enzyme**

(71) Applicant: Cargill, Incorporated, Wayzata, Minnesota 55391 (US)
(72) Inventor: The designation of the inventor has not yet been filed
(74) Representative: Wilkinson, Stephen John

(57) **Abstract**

The present invention relates to a isolated polypeptide characterised in that it comprises an amino acid sequence having at least 80% identity to a sequence selected from the group consisting of SEQ ID NO 2, SEQ ID NO 21 and SEQ ID NO 22, to polynucleotides encoding such a polypeptide and to the use thereof in the production of fructose syrups.

## Description

### Technical Field Of The Invention

The present invention relates to thermostable xylose isomerase enzymes, to polynucleotide sequences encoding such enzymes and to uses thereof.

### Background Of The Invention

Xylose isomerase (EC 5.3.1.5) is an enzyme that, *in vivo,* catalyses the reversible isomerisation of D-xylose to D-xylulose. In addition, *in vitro,* it is capable of catalysing the conversion of D-glucose to D-fructose and may thus also be referred to as a "glucose isomerase". This latter activity is used, in industry, for the production of high fructose corn syrups (HFCS).

HFCS containing 55% fructose or more has a higher sweetening power than sucrose. There is therefore an important demand for such syrups in the food and beverage industries and, as the market for HFCS increases, so does the demand for more efficient xylose isomerase enzymes.

Typically, the pH optimum of commercially available xylose isomerase enzymes ranges from about 7.5 to about 9.0. This limits the reaction temperature used in the glucose isomerisation process to around 60°C. Above this temperature, and because of non-enzymatic reactions between reducing sugars and proteins (e.g. Maillard reactions), undesirable browning products are formed.

Unfortunately, this temperature restriction does not favour high glucose conversion rates. Indeed, the isomerisation of glucose reaches an equilibrium which is shifted towards fructose at higher temperatures (Tewari, et al. (1984) J. Solut. Chem. 13, 523-547). At 60°C, syrups containing only about 40% fructose are produced. In order to produce HFCS having a greater fructose concentration, an additional chromatography step is required. It would clearly be desirable to eliminate this additional step.

Performing the isomerisation at higher temperatures (e.g. 90-95°C) would give a greater fructose concentration in HFCS. Advantageously, it could also lead to faster reaction rates, higher process stability, decreased viscosity of substrate and product streams, reduced microbial contamination and fewer problems with by-product formation. Unfortunately, however, most commercially available xylose isomerase enzymes would not be stable at such high temperatures.

Attempts have been made to obtain thermostable xylose isomerases, e.g. by site-directed mutagenesis or by screening highly thermophilic organisms for xylose isomerase activity. Both US5656497 and WO03/062387, for example, describe the isolation of a xylose isomerase enzyme from the highly thermophilic *Thermotoga neapolitana* microorganism. Xylose isomerases derived from *T. neapolitana* have been shown to be active at temperatures of 97°C. Their activity, however, is reduced to only 40% after 2 hours at 90°C and their pH optimum remains high (above 7).

As a further example, WO2004/044129 acknowledges the need for xylose isomerase enzymes which retain high levels of activity at elevated temperatures and at low pH. Referring to the experimental results shown in Figures 6-9, however, it can be seen that the peptides disclosed in this document only have optimum activity above pH 5 (see Figures 6B and 6D) and are not stable above 90°C (see Figures 8B and 8D: 50% activity lost after 1 hour and after 30 min, respectively). What is more, in order to retain high levels of activity, these enzymes require the presence of cobalt (Co²⁺) as a co-factor (see Figures 9A and 9B). Unfortunately, the use of Co²⁺ for the production of fructose syrups destined for human consumption is not recommended. Not only is it associated with a number of possible health problems, but the disposal of spent media could also contribute to environmental pollution.

The requirement for particular ions typically depends on the xylose isomerase enzyme's origin. Thus, Co²⁺ is usually required by xylose isomerases encoded by genes from thermophilic bacterial species like such as *Thermotoga neapolitana* and *Thermotoga maritima.* Possible substitutes for Co²⁺ in food-related applications include Mn²⁺ and Mg²⁺. However, these are usually only effective in combination with enzymes from non- or only slightly thermophilic organisms. It has indeed been found that the roles of Co²⁺ and Mn²⁺ or Mg²⁺ are not interchangeable. In other words, the activation of xylose isomerase from a particular source is only possible in the presence of its naturally associated co-factor (Callens, et al. (1986) Enzyme Microb. Technol. 8, 696-700; Callens, et al. (1988) Enzyme Microb. Technol. 10, 675-700; and Gaikward, et al. (1992) Enzyme Microb. Technol. 14, 317-320).

Thus, a xylose isomerase enzyme suitable for use in the food industry should ideally function under acidic conditions (i.e. at a pH of about 6 or less) to avoid browning reactions; it should and remain stable under high reaction temperatures (i.e. 85°C or more) to favour the formation of fructose; it should not require the use of potentially harmful co-factors such as Co²⁺; and it should be expressed at high levels to ease production and reduce costs.

Based on the above requirements, none of the enzymes isolated to date, whether wild-type or recombinant, would be suitable for industrial application. There remains a need, therefore, for an improved xylose isomerase with enhanced stability, activity and utility which can be produced efficiently and in quantity.

### Statements of the Invention

According to a first aspect of the present invention, there is provided an isolated polypeptide characterised in that it comprises an amino acid sequence having at least 80% identity to a sequence selected from the group consisting of SEQ ID NO 2, SEQ ID NO 21 and SEQ ID NO 22, over the entire length of said sequence, fragments and variants thereof.

According to a further aspect of the present invention, there is provided an isolated polynucleotide characterised in that it comprises a nucleotide sequence having at least 80% identity to a sequence selected from the group consisting of SEQ ID NO 1, SEQ ID NO 19 and SEQ ID NO 20, over the entire length of said sequence, fragments and variants thereof.

According to a yet further aspect of the present invention, there is provided an expression vector characterised in that it comprises a polynucleotide as defined above; and a recombinant host cell characterised in that it comprises such a vector.

According to another aspect of the present invention, there is provided a process for producing a polypeptide as defined above, comprising the steps of culturing a host cell of the invention under conditions sufficient for the production of said polypeptide and recovering the polypeptide from the culture medium.

According to yet another aspect of the present invention, there is provided an antibody that selectively binds a polypeptide as defined above.

According to a further aspect of the present invention, there is provided a method for producing a fructose syrup, comprising the step of contacting a glucose-containing composition with a polypeptide as defined above under conditions effective to convert glucose to fructose; and fructose syrup obtainable according to said method.

### Brief Description of the Drawings

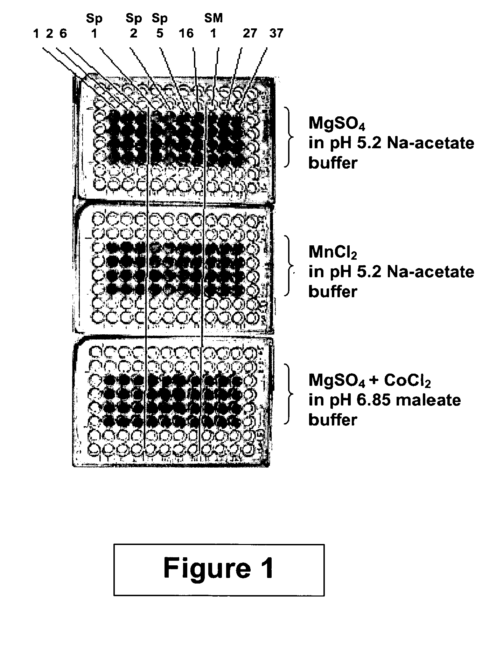

FIG. 1 - Comparison of xylose isomerase activity, with glucose as a substrate, at different pHs and in the presence of different ion co-factors. Expression of the SM-1 mutant was compared (under the same induction conditions) with that of genes cloned from wild-type species (Sp1, Sp2 + Sp5) and with that of other mutants generated by random mutagenesis (1, 2, 6, 16, 27 + 37). Genes cloned into pET22b(+) were expressed in BL21Star(DE3) cells after 1 mM IPTG induction.

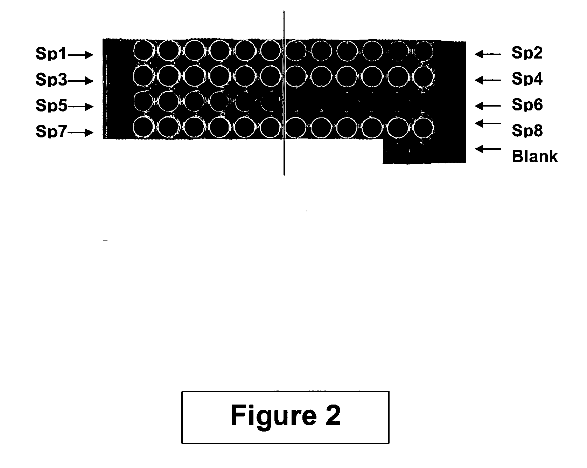

FIG. 2 - Comparison of xylose isomerase activity, with glucose as a substrate, at pH 6.85 in maleic buffer, in the presence of Mg²⁺ and Co²⁺. Genes cloned into pET22b(+) from different wild-type species (Sp1 to Sp8) were expressed in BL21Star(DE3) cells after 1 mM IPTG induction.

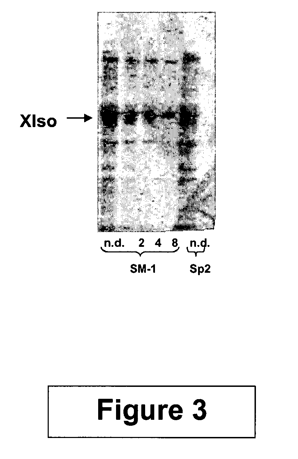

FIG. 3 - Sequential dilutions of the total protein extract from the SM-1 mutant were compared with the total protein extract from Sp2 (n.d.= not diluted; 2, 4 and 8 are the dilution factors) on a PAGE gel. All total protein extracts were obtained after induction with 1 mM IPTG. The protein band corresponding to the xylose isomerase enzyme (XIso) is indicated by an arrow.

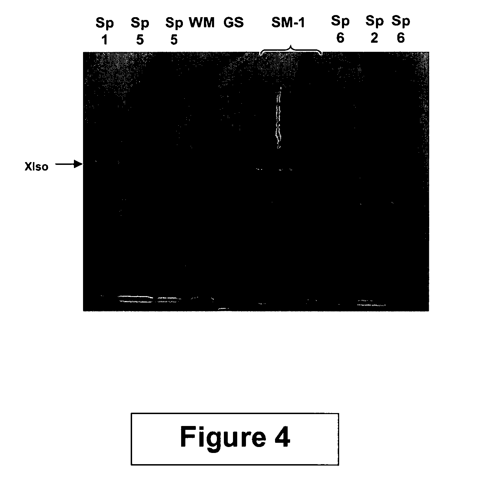

FIG. 4 - PAGE gel showing improved expression of the SM-1 mutant when compared to wild-type species (Sp1, Sp2, Sp5 and Sp6). All total protein extracts were obtained after induction with 1 mM IPTG. An aliquot of GENSWEET® SGI xylose isomerase enzyme (GS) and of Invitrogen SeeBlue® Plus 2 protein molecular weight marker (WM) were also loaded. The protein band corresponding to the xylose isomerase enzyme (XIso) is indicated by an arrow.

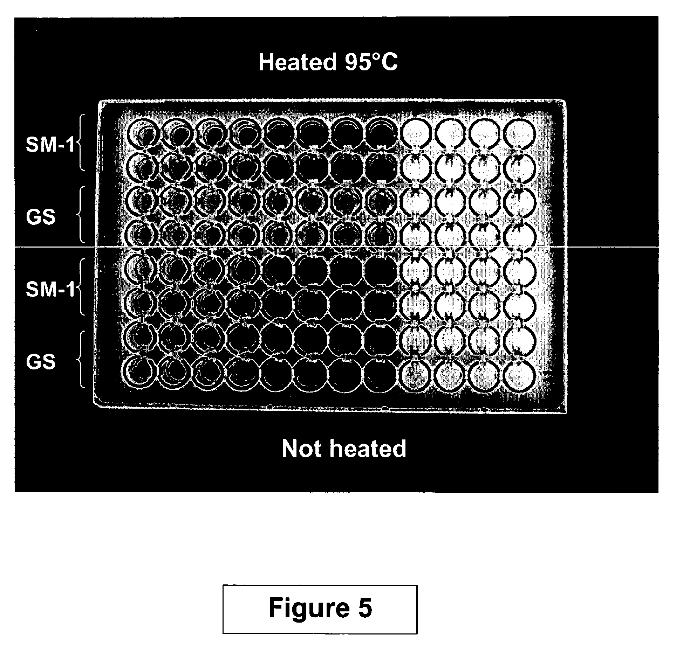

FIG. 5 - Comparison of thermo-stability between the SM-1 mutant and the commercially available GENSWEET® SGI xylose isomerase enzyme. After expression induction and heat treatment (30 min at 95°C, upper part of the plate), the test was carried out with glucose as a substrate at pH 6.85 (maleic buffer) and in the presence of Mg²⁺ and Co²⁺. Several dilutions were made. The test was repeated without heat treatment as a control. These results are shown in the lower half of the plate.

### Detailed Description Of The Invention

### Introduction

The object of the present invention, as suggested above, is to provide a thermostable xylose isomerase (or "xylose isomerase enzyme") suitable for use in the industrial or commercial production of fructose syrups.

The term "thermostable" as used herein refers to enzymes which retain at least 40 % of their activity when exposed to temperatures above 60 °C for 2 hours. Preferably, the xylose isomerase enzyme of the present invention will remain stable at temperatures in the range of from 60 to 110 °C, more preferably at temperatures in the range of from 80 to 100 °C. It may therefore also be referred to as a "hyper-thermostable" enzyme.

The enzymes of the invention will preferably also be stable under acid conditions. This means that they should retain at least 40 % of their activity when exposed to a pH of less than 8, at high temperature, for 2 hours. Preferably, the enzymes will remain stable at a pH in the range of from 4.5 to 8, more preferably in the range of from 5 to 7, even more preferably in the range of from 5 to 6.

In addition, the enzymes of the invention will preferably not require the presence of cobalt ions as co-factors for the production of fructose. More preferably, they will be capable of converting glucose to fructose using only Mn²⁺ and/or Mg²⁺ as co-factors.

Finally, when produced by standard recombinant techniques, the enzymes of the invention will preferably be expressed at higher levels than other known xylose isomerase enzymes.

These objects of the invention are met by the polypeptides and polynucleotides as defined in the attached claims and described in detail below.

### Polypeptides

In a first aspect of the present invention, there is provided a xylose isomerase polypeptide (also referred to, herein, as the "xylose isomerase enzyme"), together with biologically, industrially and commercially useful fragments and variants thereof and compositions comprising the same.

The term "polypeptide" as used herein refers to any peptide chain of more than 10, preferably of more than 100 amino acids whether in its unfolded, partially folded or fully-folded state. Fully-folded polypeptides may also be referred to as "mature" polypeptides or proteins.

The polypeptides of the invention may be "free-standing" or they may be part of a larger protein such as a precursor or a fusion protein. It may indeed be advantageous to include an additional amino acid sequence which contains secretory or leader sequences, sequences which aid in purification (such as multiple histidine residues) or additional sequences for stability during recombinant production.

In particular, the present invention provides an isolated polypeptide comprising or consisting of:
(a) an amino acid sequence which has at least 80% identity, preferably at least 85% identity, more preferably at least 90% identity, even more preferably at least 95% identity, even more preferably at least 97% identity, even more preferably at least 99% identity and most preferably exact identity to an amino acid sequence selected from the group consisting of SEQ ID NO 2, SEQ ID NO 21 and SEQ ID NO 22, over the entire length of said sequence;
(b) a polypeptide encoded by an isolated polynucleotide comprising a nucleotide sequence which has at least 80% identity, preferably at least 85% identity, more preferably at least 90% identity, even more preferably at least 95% identity, even more preferably at least 97% identity, even more preferably at least 99% identity and most preferably exact identity to a nucleotide sequence selected from the group consisting of SEQ ID NO 1, SEQ ID NO 19 and SEQ ID NO 20, over the entire length of said sequence; or
(c) a polypeptide encoded by an isolated polynucleotide comprising a nucleotide sequence having at least 80% identity, preferably at least 85% identity, more preferably at least 90% identity, even more preferably at least 95% identity, even more preferably at least 97% identity, even more preferably at least 99% identity and most preferably exact identity over its entire length to a nucleotide sequence encoding a polypeptide according to (a).

The invention further provides variants of the above defined polypeptides. In this context, the term "variant" refers to a polypeptide that differs from a reference polypeptide by the substitution, deletion and/or addition of at least one amino acid, but that otherwise retains essential properties. Generally, differences will be limited so that the sequence of the reference polypeptide and that of the variant are closely similar overall with, in many instances, large regions of exact identity.

Preferred variants in accordance with the present invention are those that have similar or the same functional characteristics as the reference polypeptide, e.g. variants comprising only silent substitutions, additions or deletions. By way of illustration, preferred variants may include conservative amino acid substitutions, whereby a residue is substituted by another with like characteristics. Examples of such substitutions include changes between Ala, Val, Leu and Ile; between Ser and Thr; between the acidic residues Asp and Glu; between Asn and Gln; between the basic residues Lys and Arg; and between the aromatic residues Phe and Tyr.

The present invention further relates to fragments of any of the above polypeptides. A fragment is a polypeptide having an amino acid sequence that is entirely the same as part (but not all) of the amino acid sequence of a reference polypeptide. Fragments may be "free-standing" or comprised within a larger polypeptide of which they form a part or region. Examples of typical fragments include truncation polypeptides, degradation polypeptides produced by or in a host cell, and fragments characterized by structural or functional attributes (such as fragments that comprise alpha-helixes, beta-sheets, hydrophilic regions, hydrophobic regions, substrate binding regions, etc.).

Preferably, the fragments of the present invention will be fragments of any of the polypeptides (or variants thereof) defined above, comprising at least 50, even more preferably at least 100 contiguous amino acids. Ideally, they will be capable of catalysing the conversion of glucose to fructose.

The homologues, variants and fragments described above will preferably retain at least 20%, more preferably at least 40%, even more preferably at least 60%, even more preferably at least 80%, even more preferably at least 90%, most preferably 100% glucose isomerase activity relative to a mature polypeptide having an amino acid sequence selected from the group consisting of SEQ ID NO 2, SEQ ID NO 21 and SEQ ID 22.

Polypeptides of the present invention can be prepared in any suitable manner including, for example, recombinant expression or synthetic production. Such methods are well known in the art.

### Antigens and Antibodies

The present invention also relates to antigens derived from the above polypeptides and to antibodies capable of reacting therewith.

An antigen (or "antigenic peptide") is a fragment of a full length polypeptide sequence (e.g. the polypeptide of SEQ ID NO 2, 21 or 22) comprising at least 6, preferably at least 10, more preferably at least 15, even more preferably at least 20, even more preferably at least 30 contiguous amino acids. It should include an epitope from that sequence (i.e. a region of the sequence which is located on the surface of the polypeptide when it is in its fully folded state) such that an antibody raised against the fragment is capable of forming a specific immune complex with the full length sequence (or with any sequence or partial sequence derived therefrom and containing the epitope).

The term "antibody" as used herein refers to molecules capable of specifically binding to such antigens, i.e. to immunoglobulin molecules, or immunologically active portions of such molecules, capable of forming an immune complex with the antigens as defined above or with polypeptides comprising such antigens. They may include, but are not limited to, polyclonal, monoclonal, chimeric and single chain antibodies and can be generated using any of a number of well known techniques (e.g. standard polyclonal or monoclonal antibody preparation techniques).

### Polynucleotides

In a further aspect of the present invention, there is provided a polynucleotide capable of encoding a xylose isomerase enzyme as defined above, together with biologically, industrially and commercially useful fragments and variants of said polynucleotide and compositions comprising the same.

The term "polynucleotide" as used herein refers to any chain of nucleotides containing at least 10, preferably at least 100 nucleotide bases. The nucleotide bases may be ribonucleotides or deoxyribonucleotides and the polynucleotides, therefore, may be either RNA or DNA molecules (whether in single- or double-stranded form). Examples of polynucleotides include, but are not limited to, unprocessed RNA, ribozyme RNA, mRNA, cDNA, genomic DNA and synthetic DNA.

In particular, the present invention provides an isolated polynucleotide comprising or consisting of:
(a) a nucleotide sequence which has at least 80% identity, preferably at least 85% identity, more preferably at least 90% identity, even more preferably at least 95% identity, even more preferably at least 97% identity, even more preferably at least 99% identity and most preferably exact identity to a polynucleotide sequence selected from the group consisting of SEQ ID NO 1, SEQ ID NO 19 and SEQ ID NO 20 over the entire length of said sequence;
(b) a nucleotide sequence encoding a polypeptide which has at least 80% identity, preferably at least 85% identity, more preferably at least 90% identity, even more preferably at least 95% identity, even more preferably at least 97% identity, even more preferably at least 99% identity and most preferably exact identity to an amino acid sequence selected from the group consisting of SEQ ID NO 2, SEQ ID NO 21 and SEQ ID NO 22, over the entire length of the said sequence;
(c) a nucleotide sequence which has at least 80% identity, preferably at least 85% identity, more preferably at least 90% identity, even more preferably at least 95% identity, even more preferably at least 97% identity, even more preferably at least 99% identity and most preferably exact identity to the polynucleotide defined in (b), over the entire length of the said sequence; or
(d) a nucleotide sequence which is complementary to, or which hybridises (preferably under stringent conditions) to, any one of the polynucleotides defined in (a)-(c) above.

For ease of reference, the polynucleotide sequences defined by SEQ ID NO 1, 19 and 20 have been represented as DNA sequences. It is of course understood that these sequences merely represent one embodiment of the present invention and that other forms of the sequences (including, for example, their RNA equivalents) also fall within the scope of the invention.

The invention further provides polynucleotides which encode any of the polypeptide variants, polypeptide fragments, precursor or fusion proteins defined above. In addition, the present invention provides variants and fragments of such polynucleotides and of the polynucleotides defined in (a)-(d) above.

In this context, the term "variant" refers to a polynucleotide that differs from a reference polynucleotide by the substitution, deletion and/or addition of at least one nucleotide, but that otherwise retains essential properties. Changes in the nucleotide sequence may or may not (e.g. because of the redundancy of the genetic code) alter the amino acid sequence of the encoded polypeptide. If changes in the nucleotide sequence do result in an alteration of the encoded amino acid sequence, this alteration will preferably not affect the functional characteristics of the resulting polypeptide (e.g. the alterations will be limited to conservative amino acid substitutions).

The term "fragments" refers to polynucleotides having a nucleotide sequence which is entirely the same as part (but not all) of the nucleotide sequence of a reference polynucleotide. Preferred fragments will comprise at least 5, more preferably at least 10, even more preferably at least 50 nucleotides and can be used, by way of example only, as primers for identifying or synthesizing full-length polynucleotide sequences. There are several methods available and well known to those skilled in the art for obtaining full-length polynucleotide sequences in this manner.

All of the above polynucleotides may be "free-standing" or they may include one or more additional nucleotide sequences. These additional sequences may be non-coding sequences (such as rho-dependent or rho-independent termination signals, ribosome binding sites, Kozak sequences, enhancer sequences, mRNA stabilising sequences, introns and poly-adenylation signals). They may also encode further amino acids (such as a marker sequence that facilitates purification of the eventual polypeptide).

Polynucleotides of the present invention can be prepared in any suitable manner including, for example, by standard cloning techniques or synthetic production (e.g. by Polymerase Chain Reaction (PCR), Ligase Chain Reaction (LCR) or Nucleic Acid Sequence-based Amplification (NASBA)). Such methods are well known in the art.

### Vectors, Host Cells and Expression Systems

The polypeptides of the present invention may be prepared by processes well known to those skilled in the art including, in particular, by genetically engineering host cells with appropriate expression vectors (i.e. with expression vectors comprising a polynucleotide of the invention).

Thus, the present invention further provides:
(a) an expression vector comprising a polynucleotide of the invention;
(b) a host cell comprising an expression vector according to (a); and
(c) a process for the production of a polypeptide of the invention by culturing a host cell according to (b) under appropriate conditions.

The term "expression vector" as used herein refers to any vector suitable for the maintenance, propagation or expression of a particular polynucleotide sequence and/or for the expression of a particular polypeptide in a chosen host. Certain vectors are capable of autonomous replication within the host while others must be integrated into, and replicated together with, the host.

Typically, the vector of the invention will be a cloning vector containing the necessary control regions (e.g. inducible or constitutive promoters) to allow for transcription and translation of the cloned polynucleotide to be initiated and regulated. The vectors will preferably also contain one or more selectable markers which permit easy selection of transformed host cells (e.g. by biocide or viral resistance or by resistance to heavy metals).

The expression vector according to the present invention may be selected from any of a great variety of suitable vectors known in the art. These include, by way of example only, chromosomal, episomal and virus-derived vectors (e.g. vectors derived from bacterial plasmids, from bacteriophage, from transposons, from yeast chromosomal elements or from viruses such as papova viruses, vaccina viruses, adenoviruses and retroviruses) and vectors derived from combinations thereof, such as those derived from plasmid and bacteriophage genetic elements (e.g. cosmids and phagemids). The choice of vector may depend on the type of host being used. Preferably, the expression vector will be a plasmid.

The polynucleotide of the invention may be inserted into the selected expression vector by any of a variety of well known and routine techniques (such as those set forth in Sambrook J., Fritsch E.F and Maniatis T. (1989) "Molecular Cloning: a Laboratory Manual" 2nd ed. Cold Spring Harbor Laboratory Press (ISBN 0-88989-509-8)).

Introduction of the expression vector into the host cell can also be achieved by any of a variety of well known methods. These include, by way of example only, calcium phosphate transfection, DEAE-dextran mediated transfection, transvection, microinjection, cationic lipid-mediated transfection, electroporation, conjugation, transduction, scrape loading, ballistic introduction and infection.

The term "host cell" is used to refer to cells capable of receiving, maintaining and allowing the reproduction of recombinant expression vectors. Preferably, the host cell will be adapted for the production of recombinant polypeptides in large quantities.

Examples of appropriate hosts include (but are not limited to): bacterial cells, such as cells of streptococci, staphylococci, enterococci, escherichia, streptomyces, cyanobacteria and bacillus; fungal cells, such as cells of a yeast, Kluveromyces, Saccharomyces, Schizosaccharomyces, Yarrowia, Pichia, a basidiomycete, Candida, Aspergillus, Acremonium, Aureobasidium, Cryptococcus, Filibasidium, Fusarium, Humicola, Magnaporthe, Mucor, Myceliophthora, Neocallimastix, Neurospora, Paecilomyces, Penicillium, Piromyces, Schizophyllum, Talaromyces, Thermoascus, Thielavia, Tolypocladium and Trichoderma; insect cells such as cells of Drosophila S2 and Spodoptera Sf9; mammalian cells such as CHO, COS, HeLa, C127, 3T3, BHK, 293, CV-1 and Bowes melanoma cells; plant cells such as cells of a gymnosperm or angiosperm; and algae cells.

Cultures of these cells are readily accessible to the public in a number of collections, such as the American Type Culture Collection (ATCC), the Deutsche Sammlung von Mikroorganismen und Zellkulturen GmbH (DSMZ), the Centraalbureau Voor Schimmelcultures (CBS), and the Agricultural Research Service Patent Culture Collection, Northern Regional Research Centre (NRRL).

Preferably, the host cell will be a bacterial or fungal cell. More preferably, the host cell will be an *E*. *coli* or *Bacillus subtilis* cell.

For production of a polypeptide according to the invention, a host cell as defined above will be cultivated under appropriate conditions. The selection of appropriate conditions will, of course, depend on the host cell being used but may include, for example, the choice of a suitable nutrient medium, cultivation temperature and pH. For each type of host cell, appropriate cultivation conditions are already well established in the art. The cell may be cultivated, by way of example only, in a shake flask or in small-scale or large-scale fermentors (including continuous, batch, fed-batch and solid state fermentors).

According to an alternative embodiment, the polypeptides of the present invention may also be translated *in vitro* as described, for example, in Zubay G. (Ann. Rev. Genet. 7, 267-287 (1973)).

Recovery and purification of the polypeptide can be achieved using any known method. These include centrifugation, filtration, extraction, spray-drying, evaporation, chromatography and precipitation. If the polypeptide is produced in a host cell and is secreted into the nutrient medium, it can be recovered directly from the medium. If the polypeptide is not secreted, it can be recovered from a cell lysate. If the polypeptide is produced in a cell-free system (i.e. *in vitro*)*,* it can be recovered directly from the reaction mixture in which it was produced.

It has surprisingly been found that, when produced under the same conditions, the polypeptides of the present invention (in particular polypeptides encoded by the polynucleotide of SEQ ID NO 1, of SEQ ID NO 19 or of SEQ ID NO 20) are expressed at levels 6 to 8 times higher than xylose isomerase sequences isolated from *Thermoanaerobacterium sp.* or *Thermotoga neapolitana.*

### Fructose Syrups

The polypeptides of the present invention - such as those obtained according to the above methods - can be used, for example, in industry for the conversion of glucose to fructose and therefore for the production of fructose syrups.

Thus, according to a further aspect of the present invention, there is provided a method for producing a fructose syrup, comprising the step of contacting a glucose-containing composition with a polypeptide as defined above, under conditions effective to convert glucose to fructose.

The fructose syrups of the present invention will preferably contain at least 30% fructose, more preferably at least 40% fructose, even more preferably at least 50% fructose by weight. According to one particular embodiment, the fructose syrup of the present invention will be a high fructose corn syrup (HFCS). High fructose corn syrups typically contain 41-43% fructose by weight. Preferably, the high fructose corn syrup of the present invention will contain at least 50%, more preferably at least 55% fructose by weight.

The glucose-containing composition used as a substrate in the above method may be any composition containing sufficient quantities of glucose to allow for the production of a fructose syrup as defined above, e.g. a glucose syrup. Preferably, however, the glucose-containing composition will be a glucose syrup comprising at least 90%, more preferably at least 95% glucose by weight.

The conversion of glucose to fructose may be carried out under standard reaction conditions as established in the art. Preferably, however, it will be carried out at a reaction temperature in the range of from 60 to 110°C, more preferably in the range of from 80 to 100°C, even more preferably in the range of from 85 to 95°C. The pH of the reaction mixture will preferably be in the range of from 4.5 to 8, more preferably 5 to 7, even more preferably 5 to 6, most preferably 5.2-5.7.

In addition to the polypeptide, the reaction mixture should also contain a xylose isomerase co-factor. This will preferably be selected from Mg²⁺, Mn²⁺ and mixtures thereof. The polypeptide may be free (e.g. added in batch or continuously) or it may be immobilised.

Fructose syrups obtained according to the above method also form part of the present invention. They may be used, for instance, in the food and beverage industries for the production of cakes, baked products, soft drinks and other consumable products.

### Advantages of the Invention

A number of advantages are associated with the products and processes of the present invention. The following is a non-exhaustive list of some of these advantages:
- the xylose isomerase enzyme of the invention is stable at high temperatures;
- it is stable at low pH;
- it does not require the use of potentially dangerous co-factors such as Co²⁺;
- it is expressed at higher levels than other xylose isomerase enzymes;
- it allows for the production of high-content fructose syrups;
- fructose syrups produced using the enzyme of the invention do not need to be further purified (e.g. by an additional chromatography step);
- browning reactions are reduced or eliminated during glucose to fructose conversion;
- reaction rates are increased;
- microbial contamination is reduced;
- etc.

### General Definitions

The term "identity" as used herein refers to the relationship between two or more polypeptide or polynucleotide sequences. In particular, it refers to the degree of sequence relatedness between those sequences. The degree of relatedness (or "percent identity") between two sequences can readily be calculated by known methods and using widely available computer programs. One example of such a method is the ClustalW method available at www.ebi.ac.uk/clustalw/index.html. For ease of reference, sequences having a certain degree of identity will be referred to herein as "homologues".

In the context of a polynucleotide sequence, the expression "identity *over the entire length of said sequence*" refers to a sequence having a certain degree of identity to a reference sequence over the entire open reading frame of that sequence.

The term "complementary" refers to a nucleic acid sequence that can form a double-stranded structure with a reference sequence. A "fully complementary" sequence is one in which each nucleic acid base is complementary to the corresponding base of its reference sequence (e.g. G to C and A to T).

In the context of hybridisation, the term "stringent conditions" means that hybridisation occurs only if there is at least 95% and preferably at least 97% identity between the sequences. Hybridisation conditions are well known in the art as exemplified in Sambrook, et al. (supra).

The term "isolated", as used herein in relation to polypeptides and polynucleotides, refers to compounds which have been changed and/or removed from their natural or original environment. For example, a polynucleotide or a polypeptide naturally present in a living organism is not "isolated". However, the same polynucleotide or polypeptide separated from the coexisting materials of its natural state is said to be "isolated". Moreover, a polynucleotide or polypeptide that is introduced to an organism e.g. by transformation or genetic manipulation is "isolated" even if it is still present in said organism.

The expression "polynucleotide encoding a polypeptide" as used herein refers to polynucleotides capable of encoding a polypeptide of the invention, either in a single continuous region or in discontinuous regions (for example, polynucleotides interrupted by an integrated phage, an integrated insertion sequence, an integrated vector sequence or an integrated transposon sequence), optionally together with additional regions that may contain coding and/or non-coding sequences.

The present invention is further described by the following example which should not be construed as limiting the scope of the invention. The example below was carried out using standard techniques, which are well known and routine to those of skill in the art, except where otherwise described in detail.

### Example: Identification of a Suitable Xylose Isomerase Polynucleotide Sequence

### Introduction

The following steps were performed to produce a polynucleotide sequence capable of encoding an xylose isomerase enzyme suitable for industrial use:
(i) Cloning a mutant xylose isomerase gene obtained as a recombinant chimera from the xylose isomerase genes of *Thermotoga neapolitana* (DSM-5068) and *Thermoanaerobacterium sp.* (DSM-8685) into a suitable plasmid expression vector and transforming this vector it into a model strain of *E. coli*;
(ii) Repeating step (i) with xylose isomerase genes from a number of wild-type strains (Sp1 to Sp8). These include the strains (Sp2 and Sp1, respectively) the xylose isomerase gene sequences of which were used as a basis for the production of the chimera of step (i);
(iii) Comparing the expression and activity of the chimeric mutant enzyme of step (i) with that of the wild-type xylose isomerase enzymes of step (ii). In terms of activity, the comparison was focused on the conversion of a glucose syrup to a fructose syrup at high temperature, low pH, and in the presence of Co²⁺, Mg²⁺ and Mn²⁺ ions (individually or in combination).
(iv) Determining the nucleotide sequence of the chimeric mutant xylose isomerase gene and predicting the corresponding amino acid sequence;
(v) Performing several rounds of random mutagenesis to improve the characteristics of the chimeric mutant xylose isomerase of step (i);
(vi) Expressing the novel mutants and screening them for the desired characteristics;
(vii) Determining the nucleotide sequence of the newly selected mutant xylose isomerase gene and deducing the amino acid sequence of the encoded enzyme.

### Materials And Methods

### • Bacterial wild-type sources of xylose isomerase genes

From DSMZ (Deutsche Sammlung von Microorganismen und Zellkulturen GmbH, Braunschweig, Germany) the following strains were purchased:
*Thermoanaerobacterium sp.* (DSM-8685), hereafter referred to as Sp1.
*Thermotoga neapolitana* (DSM-5068), hereafter referred to as Sp2.
*Thermoanaerobacterium thermosulfurigenes* (DSM-2229), hereafter referred to as Sp3.
*Thermoanaerobacterium saccharolyticum* (DSM-7060), hereafter referred to as Sp4.
*Thermotoga maritima* (DSM-3109), hereafter referred to as Sp5.
*Bacillus subtilis* (DSM-4424), hereafter referred to as Sp6.
*Thermus termophilus* (DSM-579), hereafter referred to as Sp7.

The following strain was obtained from the ARS Culture Collection at National Centre for Agricultural Utilization Research (Peoria, Illinois, USA):
*Arthrobacter* (NRRL-B3728), hereafter referred to as Sp8.

Growth conditions: strains Sp1 to Sp5 were grown under anaerobic conditions; strains Sp6, Sp7 and Sp8 were grown under aerobic conditions. Growth media and temperature conditions for the DSM strains were the ones suggested by the DSMZ website (http://www.dsmz.de/strains). Nutrient agar and broth were used for the growth of Sp8.

### • Bacterial host strains for plasmid transformation

*Escherichia coli* TOP10 (Invitrogen) and BL21 Star^{™} (DE3) (Invitrogen) were used as host strains for cloning and expression purposes, respectively. Growth was achieved in liquid or solid plates in standard Luria-Bertani (LB) medium supplemented, when necessary, with 100 µg/ml carbenicillin (SIGMA)

### • Genomic DNA extraction

Approximately 300 mg of bacterial pellet obtained from bacterial cultures of species Sp1 to Sp8 were subjected to genomic DNA extraction by means of the "Wizard® Magnetic - DNA Purification System For Food" kit (Promega). Cell disruption was accomplished mechanically by means of stainless steel beads and a Beat Beater. DNA was eluted in ddH₂O, quantified by standard gel electrophoresis and an aliquot directly subjected to PCR.

### • Primers design

DNA sequences of xylose isomerase genes from species Sp1 to Sp8 were obtained from Genbank. Forward and reverse PCR primers were designed to be complementary to the end moieties of each gene. A 5' tail, encoding sequences upstream of the NdeI and downstream of the SacI restriction sites of pET22b(+) were added to the forward and reverse primers, respectively. Reverse primers were designed to contain a double stop codon before the poly-histidine tract encoded by the vector. These tails described above were later used as "universal" forward and reverse primers, e.g. to rescue and further clone genes subjected to random mutagenesis.

Primers were designed using thermodynamic predictions (see, for example, Tinoco, et al. (1973) Nature New Biol. 246, 40-41; Gralla, et al. (1973) J. Mol. Biol. 73, 497-511; Papanicolau, et al. (1983) Nucleic Acids Res. 12, 31-44; and Freier, et al. (1986) Proc. Natl. Acad. Sci. 83, 9373-9377) and in-house software. The following primers were prepared:

| **Primer Name** | **5'-3' Sequence** |
|---|---|
| XIsoSp1-Nde-F | SEQ ID NO 3 |
| XIsoSp1-Sac-R | SEQ ID NO 4 |
| XIsoSp2-Nde-F | SEQ ID NO 5 |
| XIsoSp2-Sac-R | SEQ ID NO 6 |
| XIsoSp3&4-Nde-F | SEQ ID NO 7 |
| XIsoSp3&4-Sac-R | SEQ ID NO 8 |
| XIsoSp5-Nde-F | SEQ ID NO 9 |
| XIsoSp5-Sac-R | SEQ ID NO 10 |
| XIsoSp6-Nde-F | SEQ ID NO 11 |
| XIsoSp6-Sac-R | SEQ ID NO 12 |
| XIsoSp7-Nde-F | SEQ ID NO 13 |
| XIsoSp7-Sac-R | SEQ ID NO 14 |
| XlsoSp8-Nde-F | SEQ ID NO 15 |
| XlsoSp8-Sac-R | SEQ ID NO 16 |
| Universal-Nde-F | SEQ ID NO 17 |
| Universal-Sac-R | SEQ ID NO 18 |

| | |
|---|---|
| Note: the same primers were designed for amplification of the xylose isomerase genes of both Sp3 and Sp4 as they share common terminal sequences. | |

### • Amplification and cloning of xylose isomerase genes

The PCR reaction mixture, in a volume of 50 µl, contained 10 ng of genomic DNA, 15 pmol of each primer, 10 µl of Platinum® Pfx Amplification Buffer, 0.3 mM each dNTP, 1 mM MgSO₄, 1.25 Units Platinum® Pfx DNA polymerase (Invitrogen). The thermal profile consisted of an initial denaturation step of 5 min at 94°C, followed by 50 cycles consisting of 94°C for 15 sec, an annealing temperature between 54 and 64°C (depending on the primer couple) for 30 sec, and 68°C for 2 min.

Amplification of the 1.4-kb product was checked by running a small aliquot of the reaction on a 1.3% TAE agarose gel. The PCR product was purified using either QIAquick® PCR Purification kit or QIAquick® Gel Extraction kit (QIAGEN) and cloned back into the NdeI and SacI sites of pET22b(+). Restriction enzymes used were purchased from either Promega, Fermentas or New England BioLabs. DNA digestion with restriction endonucleases, separation of fragments by agarose gel electrophoresis, vector dephosphorylation with Shrimp Alkaline Phosphatase (Promega) and ligation of DNA fragments with T4 DNA ligase (New England BioLabs) were accomplished with standard molecular biology techniques (Sambrook, et al. (supra)), and following manufacturers' recommendations (with slight modifications where necessary).

Transformation into E. *coli* BL21 Star^{™} (DE3) cells of the xylose isomerase genes cloned into pET22b(+) was carried out through electroporation. Cell preparation was accomplished following the protocols described in Ausubel I. et al. ("Current Protocols in Molecular Biology" (1994-2000) John Wiley Sons Inc. (ISBN 0-471-50338-X)). Approximately 150 µl of cell suspension were electroporated in 2 mm electrode gap cuvettes by means of a Gene Pulser® II device (Bio-Rad). Set working parameters were 2.5 KV, 25 µF and 200 Ohm.

### • Sequence analysis and comparison

The following programs were used to align the sequences (available from Genbank) of several xylose isomerase genes:
ClustalW (www.ebi.ac.uk/clustalw/index.html);
NCBI Blast2 (www.ebi.ac.uk/blastall/index.html); and
EMBOSS (http://emboss.sourceforge.net).

This alignment provided a tool to predict wanted characteristics and to identify possible stable mRNA secondary structures which can inhibit translation initiation and correct termination.

### • Production of a chimeric mutant

Based on the above alignment, and without disrupting the correct reading frame, a BclI restriction site was introduced into the xylose isomerase genes from Sp2 and Sp1 using engineered PCR primers.

By means of PCR, a DNA fragment encompassing the complete 5' moiety of the xylose isomerase gene of Sp2 and the BclI site in its 3' terminal moiety was generated. Another fragment encompassing the complete 3' moiety of xylose isomerase of Sp 1 and the BclI site in its 5' terminal moiety was also generated. Platinum® Pfx DNA polymerase (Invitrogen) was employed in the same conditions described above to achieve these amplifications at an annealing temperature of 56°C. DNA products were purified by means of a QIAquick® Gel Extraction kit. 500 ng of each amplified fragment were then cut with 20 Units of BclI (Fermentas) for 3 hours at 55°C.

The desired restriction products were purified from the gel as described above and an aliquot thereof was ligated. A total of 100 ng DNA were put into reaction in a final volume of 20 µl. After an overnight incubation at 16°C, the reaction was stopped by heating for 10 min at 70°C, set immediately on ice and purified by means of QIAquick® PCR Purification kit. Ligated products were rescued using the "universal primers" and the PCR protocol described above.

An amplification product of the expected size was purified by means of the QIAquick® Gel Extraction kit, digested with NdeI and SacI and cloned into the respective restriction sites of pET22b(+). Transformation was accomplished through electroporation into either TOP10 or BL21 Star^{™} (DE3) E. *coli* cells and plated overnight on Luria-Bertani (LB) agar plates containing 100ug/ml carbenicillin.

### • Random mutagenesis

Random mutations were introduced into the xylose isomerase chimeric gene mutant cloned into the NdeI and SacI restriction sites of pET22b (+). PCR was performed with the "universal primers" described above. The reaction mixture, according to the protocol proposed by Cadwell and Joyce (1992 - PCR Meth. Appl. 2, 28-33), contained 2 ng chimeric gene DNA, 30 pmol of each primer, 50 mM KCI, 10 mM Tris-HCl (pH 8.3), 7.0 mM MgCI₂, 0,5 mM MnCI₂,1 mM dCTP, 1 mM dTTP, 0.2 mM dATP, 0.2 mM dGTP, and 2.5 Units AmpliTaq DNA polymerase (Applied Biosystems) in a 50 µl reaction volume. After an initial denaturation step of 3 min at 94°C, cycling parameters were made of 40 cycles of 94°C for 30 sec, 54°C for 30 sec, and 72°C for 1 min.

Amplification of the 1.4-kb product was checked by running the reaction on a 1.3 % agarose gel. The PCR product was purified using QIAquick® Gel Extraction kit and ligated back into the NdeI and SacI sites of dephosphorylated pET22b (+), as described above.

For the following rounds of random mutagenesis, genes from selected mutants were used as template. Three rounds of random mutagenesis were carried out. Each time a library of approximately 10,000 samples was screened, the best candidate was selected and subjected to a further cycle of mutagenesis.

Transformation was accomplished through electroporation into BL21 Star^{™} (DE3) E. *coli* cells and plated overnight on Luria-Bertani (LB) agar plates containing 100µg/ml carbenicillin.

### • Oligonucleotide synthesis and DNA sequencing

PCR primer synthesis and DNA sequencing were accomplished by MWG (Ebersberg, Germany).

### • Culture of recombinant clones in 96 well plates, thermal cell lysis and test for glucose isomerisation

Single colonies were transferred into 96-well plates, each well containing 100 µl of LB and carbenicillin at a final concentration of 100 µg/ml. Plates were incubated overnight at 37°C on an orbital shaker at 200 rpm to allow cell growth. From each well 55 µl culture were transferred to another plate. The mother plate was stored at +4°C to save the original clones. To the daughter plate 5 µl of a IPTG solution in LB substrate were added, so that a final concentration of 1 mM was reached for IPTG. The plate was further incubated for 8 hours at 37°C on an orbital shaker at 200 rpm. A thermal step of 30 min at 95°C was carried out, then 60 µl of buffer solution including glucose as a substrate (see below) were added. Plates were sealed and placed for 20-24 h in an oven at 60°C. The sulphuric acid method proposed by Kennedy and Chaplin (1975 - Carbohydrate Res. 40, 227-233) was used to quantitatively detect D-fructose: after having cooled them down at room temperature, 120 µl of 66% sulphuric acid were added, each plate was tightly sealed and incubated at 60°C for 30-60 min. Colour development was determined by reading on a micro-plate reader (BMG) using a 405 nm filter. Samples selected over a defined threshold were recovered from the mother plate and further investigated after having grown them in a higher volume (see below). Crude extracts of BL21 Star^{™}(DE3) cells harbouring an "empty" pET22b(+) or xylose isomerase genes cloned from Sp1 to Sp8 into pET22b(+) were used as reference controls on each plate. All operations involving micro-plates were carried out manually or by means of a HT robotic workstation (Beckman-Coulter).

### • Culture of recombinant clones in 50 ml tubes, enzymatic cell lysis and test for glucose isomerisation.

A single colony-derived cell pre-culture was cultivated overnight in a volume of 3 ml LB containing a final concentration of 100 µg/ml carbenicillin. One hundred micro-liters were inoculated into 50-ml polypropylene tubes containing 10 ml LB medium containing 100 µg/ml carbenicillin and left to grow for 20 hours at 37°C on an orbital shaker at 250 rpm. IPTG was added to reach a final concentration of 1 mM, growth was continued for 8 hours at 37°C on an orbital shaker at 250 rpm.

The supernatant was discarded, the pellet was lysed by adding BugBusterHT® (Novagen), 1/5 with respect to the culture volume, plus rLysozyme^{™} (Novagen), 1/75 of the culture volume. The latter component was dissolved just before use 1:750 (w/v) in 50 mM TrisCl pH 7.8. Lysis was carried out for 30 min at room temperature. Tubes were shaken manually from time to time. The samples were centrifuged for 10 min at 4200 xg at room temperature, then 60 µl of the supernatant were transferred to a 96-well micro-plate. A thermal step of 30 min at 95°C was carried out, then 60 µl of substrate plus buffer solution were added. Plates were sealed and placed for 20-24 h in an oven at 60°C. After having cooled down the plates at room temperature, 120 µl of 66% sulphuric acid were added, the plates were tightly sealed and incubated at 60°C for 30-60 min. Color development was determined by reading on a micro-plate reader (BMG) using a 405 nm filter.

The same enzyme activity controls as described above were used. A commercial xylose isomerase preparation like GENSWEET® SGI (Genencor) was used as a reference control.

### • Buffer conditions used for enzymatic test

Enzymatic tests were carried out in 100 mM maleic acid buffer pH 6.85 or in 50 mM Sodium-acetate buffer pH 5.2. Final pH values revealed to be increased of about 0.5-0.6 pH units after the addition of the crude cell lysis extracts. D-glucose was added as a substrate at a final concentration of 12.5%. The effect of metal cations was established by adding MgSO₄ at a final concentration of 10 mM, CoCl₂ at a final concentration of 0.5 mM and MnCl₂ at a final concentration of 5 mM. The effect of the ions was evaluated either individually or in combinations (MgSO₄ + CoCl₂ and MgSO₄ + MnCl₂).

### • PAGE analysis of xylose isomerase mutants expression level

Cell cultures and expression induction were performed as indicated above, with the following modifications:

50-ml polypropylene tubes containing 10 ml LB medium + 100 µg/ml carbenicillin were inoculated from single colonies and left to grow for 48-72 hours at 37°C on an orbital shaker at 250 rpm. IPTG was added to reach a final concentration of 1 mM, and the induction step was carried out for about 24 hours at 37°C, on an orbital shaker at 250 rpm. 5 milliliters were centrifuged for 20 min at 4200 xg at +4°C. The supernatant was discarded, 1 ml of BugBusterHT ® (Novagen), and 70 µl of rLysozyme^{™} (Novagen), the latter dissolved just before use (1:750 (w/v) in 50 mM TrisCl pH 8.0), were added to the pellet. After vortexing, lysis was carried out for 25 min at room temperature. Tubes were shaken periodically by hand. The samples were centrifuged again at 4200 xg at 4°C for 20 min. Crude extracts, i.e. the supernatants, containing the soluble protein fraction to be investigated, were stored at +4°C.

For the PAGE analysis pre-cast, NuPAGE® Novex Bis-Tris gels (Invitrogen), 10% polyacrylamide, 1 mm width, containing 10-wells or 15-wells were used in a Xcell SureLock^{™} Mini-Cell for protein electrophoresis (Invitrogen). On ice, 3 or 2 µl crude extracts were aliquoted into 1.5 ml polypropylene tubes. A volume of 6.5 µl was reached with ddH₂O. 1 µl of NuPAGE® Reducing Agent 10x (Invitrogen) and 2.5 µl of NuPAGE® LDS Sample Buffer 4x (Invitrogen) were added to a final volume of 10 µl. Samples were mixed with a pipette and spun down. The tubes were tightly closed and the samples denatured for 10 min in a water bath set at 70°C.

Electrophoresis was carried out in NuPAGE® MOPS SDS Running Buffer 1x (Invitrogen). 200 ml of buffer contained inside the inner cell chamber were supplemented with 500 µl of NuPAGE® Reducing Agent 10x (Invitrogen). Between 2 to 5 µl of denatured samples were loaded with the aid of a micropipette. 3 µl of SeeBlue® Plus2 Pre-Stained Standard (Invitrogen) were used as a protein molecular mass standard. The expected molecular mass of the xylose isomerase genes is about 50 kDa. The electrophoretic run was carried out at 200 constant Volts for 50 min, according to manufacturer's instruction. Each gel was stained in 20 ml of SimplyBlue^{™} SafeStain (Invitrogen) for 1 hour, under light agitation and de-stained in distilled water for approximately 1 hour.

### Results and Discussion

The best xylose isomerase mutant, hereafter named SM-1 for sake of clarity, is encoded by the sequence given in SEQ ID NO 1 (where the BclI site introduced to join the original Sp1 and Sp2 fragments - as described above - is underlined) and its deduced protein sequence is given in SEQ ID NO 2. The mutant was expressed intracellularly, in the cytoplasm, the pe1B leader peptide coded in between the NdeI and SacI restriction sites of the pET22b(+) vector having been removed. This and all other mutants were produced in their native-like conformation, i.e. without any amino- or carboxyl-terminal extensions. A comparison of performance between SM-1 and the xylose isomerase enzymes coded by genes from other species (Sp1 to Sp8), also cloned in the system pET22b(+)/BL21 Star ^{™}(DE3), was carried out. In particular, the enzymatic activity was monitored in different buffer conditions and in the presence of different ion combinations (FIG. 1 and FIG. 2). A further investigation was carried out on the expression level through PAGE analysis (FIG. 3 and FIG. 4).

The enzymatic performance was tested at a temperature of 60°C but a previous thermal step of 30 min at 95°C was done not only to lyse the bacterial cells when grown in micro-plates, but also to eliminate the background of non-thermal resistant proteins and of mutated xylose isomerase enzymes with inefficient thermal behaviour. It is clear from the data shown that in these conditions the selected mutant SM-1 shows a higher thermal resistance in comparison to a commercial xylose isomerase enzyme like GENSWEET® SGI (FIG. 5). The mutant's thermal resistance is very similar to the one shown by hyper-thermophilic xylose isomerase enzymes from *T. neapolitana* (Sp2) and *T. maritima* (Sp5). The enzyme performance with glucose as a substrate was higher when compared to that of other highly thermophilic species at pH 7.5 in the presence of Mg²⁺ and Co²⁺, and much higher in the presence of Mg²⁺ and/or Mn²⁺ (alone or in combination) at pH 5.7 (FIG. 1).

Protein expression of the chosen mutant SM-1 is also consistently higher (8-10 times) in comparison to other species and, in particular, to the species *Thermoanaerobacterium sp.* (Sp1) and *T. neapolitana* (Sp2) used as original parents for the initial chimera construction (FIG. 4).

For these reasons, the xylose isomerase enzyme produced by the selected mutant SM-1 can be used for an efficient conversion of glucose into fructose at high temperature, low pH and without the presence of Co²⁺ ions. The higher level of expression of the SM-1 gene will allow for cheaper and more convenient enzyme preparation.

### Annex to the application documents - subsequently filed sequences listing

SEQUENCE LISTING

<110> Cargill, Incorporated

<120> Thermostable Xylose Isomerase Enzymes

<130> SJW/9294 EP

<140> EP 06010465.0
<141> 2006-05-20

<160> 22

<170> PatentIn version 3.3

<210> 1
<211> 1320
<212> DNA
<213> Artificial sequence

<220>
<221> source
<223> /note= "Description of artificial sequence: Polynucleotide encoding
a xylose isomerase polypeptide"

<400> 1
atggctgaat tctttccaga aatcccgaaa gtgcagttcg aaggcaaaga aagcacaaat 60

ccacttgcgt tcaagttcta cgatccagaa gagatcatcg acggcaaacc cctcaaggac 120

catctgaagt tctccgttgc cttctggcac accttcgtga acgagggaag ggatcccttc 180

ggagacccaa cggccgatcg tccatggaac aggtacaccg atccaatgga caaggctttt 240

gcaagggtgg acgccctttt tgaattctgc gaaaaactca acatcgagta cttctgcttc 300

cacgacagag acatcgctcc cgagggaaaa acgctgaggg agacaaacaa aattttggac 360

aaagtagtgg agagaatcaa agagagaatg aaagacagca acgtgaagct cctctggggt 420

actgcaaacc tcttttccca cccaaggtac atgcatggtg cagcgacaac ctgcagtgct 480

gatgtttttg cgtacgcggc cgcccaggtg aaaaaggccc ttgagatcac caaagaactt 540

ggaggagaag ggtacacatc ctggggtgga agagaaggat acgaaacact cctcaacacg 600

gaccttggat tcgaacttga aaacctcgcc cgcttcctca gaatggctgt ggattatgca 660

aaaaggatcg gtttcaccgg acagttcctc atcgaaccaa aaccgaaaga acccaccaaa 720

caccagtacg acttcgacgt tgcaaccgcc tatgccttcc tgaagagcca cggtctagat 780

gagtacttca agttcaacat tgaagcgaac cacgcgacac ttgccggtca cacattccag 840

cacgaactga ggatggcaag gatcctcgga aaacttggaa gcatcgacgc caaccaggga 900

gatctcctgc tcggctggga caccgaccag ttcccaacga acatctacga cacaacactt 960

gccatgtacg aagtgatcaa gatgggggga tttgacaaag gcggccttaa cttcgatgca 1020

aaagtaagac gtgcttcatt tgagccagaa gatcttttct taggtcacat agctggaatg 1080

gattcttttg caaaaggctt caaggttgct tacaagcttg tgaaagatgg cgttttcgac 1140

aagtttatcg aggaaagata cgcaagctac aaagatggca ttggcgctga tattgtaagt 1200

ggaaaggctg acttcaagag ccttgagaag tatgcattag agcacagcca gattgtcaac 1260

aaatcaggaa gacaagagct attggagtca atcctaaatc agtatttgtt tgcagaatga 1320

<210> 2
<211> 439
<212> PRT
<213> Artificial sequence

<220>
<221> source
<223> /note= "Description of artificial sequence: Polypeptide sequence
encoded by SEQ ID NO: 1"

<400> 2

Met Ala Glu Phe Phe Pro Glu Ile Pro Lys Val Gln Phe Glu Gly Lys
1               5                   10                  15

Glu Ser Thr Asn Pro Leu Ala Phe Lys Phe Tyr Asp Pro Glu Glu Ile
            20                  25                  30

Ile Asp Gly Lys Pro Leu Lys Asp His Leu Lys Phe Ser Val Ala Phe
        35                  40                  45

Trp His Thr Phe Val Asn Glu Gly Arg Asp Pro Phe Gly Asp Pro Thr
    50                  55                  60

Ala Asp Arg Pro Trp Asn Arg Tyr Thr Asp Pro Met Asp Lys Ala Phe
65                  70                  75                  80

Ala Arg Val Asp Ala Leu Phe Glu Phe Cys Glu Lys Leu Asn Ile Glu
                85                  90                  95

Tyr Phe Cys Phe His Asp Arg Asp Ile Ala Pro Glu Gly Lys Thr Leu
            100                 105                 110

Arg Glu Thr Asn Lys Ile Leu Asp Lys Val Val Glu Arg Ile Lys Glu
        115                 120                 125

Arg Met Lys Asp Ser Asn Val Lys Leu Leu Trp Gly Thr Ala Asn Leu
    130                 135                 140

Phe Ser His Pro Arg Tyr Met His Gly Ala Ala Thr Thr Cys Ser Ala
145                 150                 155                 160

Asp Val Phe Ala Tyr Ala Ala Ala Gln Val Lys Lys Ala Leu Glu Ile
                165                 170                 175

Thr Lys Glu Leu Gly Gly Glu Gly Tyr Thr Ser Trp Gly Gly Arg Glu
            180                 185                 190

Gly Tyr Glu Thr Leu Leu Asn Thr Asp Leu Gly Phe Glu Leu Glu Asn
        195                 200                 205

Leu Ala Arg Phe Leu Arg Met Ala Val Asp Tyr Ala Lys Arg Ile Gly
    210                 215                 220

Phe Thr Gly Gln Phe Leu Ile Glu Pro Lys Pro Lys Glu Pro Thr Lys
225                 230                 235                 240

His Gln Tyr Asp Phe Asp Val Ala Thr Ala Tyr Ala Phe Leu Lys Ser
                245                 250                 255

His Gly Leu Asp Glu Tyr Phe Lys Phe Asn Ile Glu Ala Asn His Ala
            260                 265                 270

Thr Leu Ala Gly His Thr Phe Gln His Glu Leu Arg Met Ala Arg Ile
        275                 280                 285

Leu Gly Lys Leu Gly Ser Ile Asp Ala Asn Gln Gly Asp Leu Leu Leu
    290                 295                 300

Gly Trp Asp Thr Asp Gln Phe Pro Thr Asn Ile Tyr Asp Thr Thr Leu
305                 310                 315                 320

Ala Met Tyr Glu Val Ile Lys Met Gly Gly Phe Asp Lys Gly Gly Leu
                325                 330                 335

Asn Phe Asp Ala Lys Val Arg Arg Ala Ser Phe Glu Pro Glu Asp Leu
            340                 345                 350

Phe Leu Gly His Ile Ala Gly Met Asp Ser Phe Ala Lys Gly Phe Lys
        355                 360                 365

Val Ala Tyr Lys Leu Val Lys Asp Gly Val Phe Asp Lys Phe Ile Glu
    370                 375                 380

Glu Arg Tyr Ala Ser Tyr Lys Asp Gly Ile Gly Ala Asp Ile Val Ser
385                 390                 395                 400

Gly Lys Ala Asp Phe Lys Ser Leu Glu Lys Tyr Ala Leu Glu His Ser
                405                 410                 415

Gln Ile Val Asn Lys Ser Gly Arg Gln Glu Leu Leu Glu Ser Ile Leu
            420                 425                 430

Asn Gln Tyr Leu Phe Ala Glu
        435

<210>  3
<211>  62
<212>  DNA
<213>  Artificial sequence

<220>
<221> source
<223> /note= "Description of artificial sequence: Primer XIsoSp1-Nde-F"

<400>  3
atttgtttta actttaagaa ggagatatac atatgaataa atattttgag aatgtatcta      60

aa                                                                      62

<210>  4
<211>  45
<212>  DNA
<213>  Artificial sequence

<220>
<221> source
<223> /note= "Description of artificial sequence: Primer XIsoSp1-Sac-R"

<400>  4
agcttgtcga cgagctcttt atcattctgc aaacaaatac tgatt                      45

<210> 5
<211> 54
<212> DNA
<213> Artificial sequence

<220>
<221> source
<223> /note= "Description of artificial sequence: Primer XIsoSp2-Nde-F"

<400> 5
attttgttta actttaagaa ggagatatac atatggctga attctttcca gaaa 54

<210> 6
<211> 43
<212> DNA
<213> Artificial sequence

<220>
<221> source
<223> /note= "Description of artificial sequence: Primer XIsoSp2-Sac-R"

<400> 6
agcttgtcga cgagctcttt atcacctcag ttccagaatg gtc 43

<210> 7
<211> 62
<212> DNA
<213> Artificial sequence

<220>
<221> source
<223> /note= "Description of artificial sequence: Primer XIsoSp3&4-Nde-F"

<400> 7
attttgttta actttaagaa ggagatatac atatgaataa atattttgag aacgtatcta 60

aa 62

<210> 8
<211> 48
<212> DNA
<213> Artificial sequence

<220>
<221> source
<223> /note= "Description of artificial sequence: Primer XIsoSp3&4-Sac-R"

<400> 8
agcttgtcga cgagctcttt attattctgc aaacaaatac tgatttag 48

<210> 9
<211> 54
<212> DNA
<213> Artificial sequence

<220>
<221> source
<223> /note= "Description of artificial sequence: Primer XIsoSp5-Nde-F"

<400> 9
attttgttta actttaagaa ggagatatac atatggcaga atttttccca gaaa 54

<210> 10
<211> 46
<212> DNA
<213> Artificial sequence

<220>
<221> source
<223> /note= "Description of artificial sequence: Primer XIsoSp5-Sac-R"

<400> 10
agcttgtcga cgagctcttt atcacctcag ttctgctatt gtcttc 46

<210> 11
<211> 53
<212> DNA
<213> Artificial sequence

<220>
<221> source
<223> /note= "Description of artificial sequence: Primer XIsoSp6-Nde-F"

<400> 11
attttgttta actttaagaa ggagatatac atatggctca atctcattcc agt 53

<210>  12
<211>  51
<212>  DNA
<213>  Artificial sequence

<220>
<221> source
<223> /note= "Description of artificial sequence: Primer XIsoSp6-Sac-R"

<400>  12
agcttgtcga cgagctcttt attatacttc taaaatgtat tggttcaata t          51

<210>  13
<211>  52
<212>  DNA
<213>  Artificial sequence

<220>
<221> source
<223> /note= "Description of artificial sequence: Primer XIsoSp7-Nde-F"

<400>  13
attttgttta actttaagaa ggagatatac atatgtacga gcccaaaccg ga          52

<210>  14
<211>  39
<212>  DNA
<213>  Artificial sequence

<220>
<221> source
<223> /note= "Description of artificial sequence: Primer XIsoSp7-Sac-R"

<400>  14
agcttgtcga cgagctcttt atcacccccg cacccccag          39

<210>  15
<211>  50
<212>  DNA
<213>  Artificial sequence

<220>
<221> source
<223> /note= "Description of artificial sequence: Primer XIsoSp8-Nde-F"

<400> 15
attttgttta actttaagaa ggagatatac atatgagcgt tcagccgacc 50

<210> 16
<211> 39
<212> DNA
<213> Artificial sequence

<220>
<221> source
<223> /note= "Description of artificial sequence: Primer XIsoSp8-Sac-R"

<400> 16
agcttgtcga cgagctcttt attagcggga gccgagcag 39

<210> 17
<211> 35
<212> DNA
<213> Artificial sequence

<220>
<221> source
<223> /note= "Description of artificial sequence: Primer Universal-Nde-F"

<400> 17
attttgttta actttaagaa ggagatatac atatg 35

<210> 18
<211> 21
<212> DNA
<213> Artificial sequence

<220>
<221> source
<223> /note= "Description of artificial sequence: Primer Universal-Sac-R"

<400> 18
agcttgtcga cgagctcttt a 21

<210> 19
<211> 1320
<212> DNA
<213> Artificial sequence

<220>
<221> source
<223> /note= "Description of artificial sequence: Polynucleotide encoding
      a xylose isomerase polypeptide"

<400> 19
atggctgaat tctttccaga aatcccgaaa gtacagttcg aaggcaaaga aagcacaaat      60

ccacttgcgt tcaagttcta cgatccagaa gaggtcatcg acggcaaacc cctcaaggac     120

catctgaagt tctccgttgc cttctggcac accttcgtga acgagggaag ggatcccttc     180

ggagacccaa cggccgatcg tccatggaac aggtacaccg atccaatgga caaggctttt     240

gcaagggtgg acgccctttt tgaattctgc gaaaaactca acatcgagta cttctgcttc     300

cacgacagag acatcgctcc cgagggaaaa acgctgaggg agacaaacaa aattttggac     360

aaagtagtgg agagaatcaa agagagaatg aaagacagca acgtgaagct cctcttgggt     420

actgcaaacc tcttttccca cccaaggtac atgcatggtg cagcgacaac ctgcagtgct     480

gatgtttttg cgtacgcggc cgcccaggtg aaaaaggccc ttgagatcac caaagaactt     540

ggaggagaag ggtacgtatt ctggggtgga agagaaggat acgaaacact cctcaacacg     600

gaccttggat tcgaatttga aaacctcgcc cgcttcctca gaatggctgt ggattatgca     660

aaaaagatcg gtttcaccgg acagttcctc atcgaaccaa aaccgaaaga acccaccaaa     720

caccagtacg acttcgacgt tgcaaccgcc tatgccttcc tgaagagcca cggtctagat     780

gagtacttca agttcaacat tgaagcgaac cacgcgacac ttgccggtca cacattccag     840

cacgaactga ggatggcaag gatcctcgga aaacttggaa gcatcgacgc caaccaggga     900

gatctcctgc tcggctggga caccgaccag ttcccaacga acgtctacga cacaacactt     960

gccatgtacg aagtgatcaa gatgggggga tttgacaaag gcggccttaa cttcgatgca    1020

aaagtaagac gtgcttcatt tgagccagaa gatcttttct taggtcacat agctggaatg 1080

gattcttttg caaaaggctt caaggttgct tacaagcttg tgaaagatgg cgttttcgac 1140

aagtttatcg aggaaagata cgcaagctac aaagatggca ttggcgctga tattgtaagt 1200

ggaaaggctg acttcaagag ccttgagaag tatgcattag agcacagcca gattgtcaac 1260

aaatcaggaa gacaagagct attggagtca atcctaaatc agtatttgtt tgcagaatga 1320

<210> 20
<211> 1320
<212> DNA
<213> Artificial sequence

<220>
<221> source
<223> /note= "Description of artificial sequence: Polynucleotide encoding
      a xylose isomerase polypeptide"

<400> 20
atggctgaat tctttccaga aatcccgaaa gtacagttcg aaggcaaaga aagcacaaat 60

ccacttgcgt tcaagttcta cgatccagaa gaggtcatcg acggcaaacc cctcaaggac 120

catctgaagt tctccgttgc cttctggcac accttcgtga acgagggaag ggatcccttc 180

ggagacccaa cggccgatcg tccatggaac aggtacaccg atccaatgga caaggctttt 240

gcaagggtgg acgccctttt tgaattctgc gaaaaactca acatcgagta cttctgcttc 300

cacgacagag acatcgctcc cgagggaaaa acgctgaggg agacaaacaa aattttggac 360

aaagtagtgg agagaatcaa agagagaatg aaagacagca acgtgaagct cctcttgggt 420

actgcaaacc tcttttccca cccaaggtac atgcatggtg cagcgacaac ctgcagtgct 480

gatgtttttg cgtacgcggc cgcccaggtg aaaaaggccc ttgagatcac caaagaactt 540

ggaggagaag ggtacacatc ctggggtgga agagaaggat acgaaacact cctcaacacg 600

gaccttggat tcgaatttga aaacctcgcc cgcttcctca gaatggctgt ggattatgca 660

aaaaagatcg gtttcaccgg acagttcctc atcgaaccaa aaccgaaaga acccaccaaa 720

caccagtacg acttcgacgt tgcaaccgcc tatgccttcc tgaagagcca cggtctagat    780

gagtacttca agttcaacat tgaagcgaac cacgcgacac ttgccggtca cacattccag    840

cacgaactga ggatggcaag gatcctcgga aaacttggaa gcatcgacgc caaccaggga    900

gatctcctgc tcggctggga caccgaccag ttcccaacga acgtctacga cacaacactt    960

gccatgtacg aagtgatcaa gatgggggga tttgacaaag gcggccttaa cttcgatgca   1020

aaagtaagac gtgcttcatt tgagccagaa gatcttttct taggtcacat agctggaatg   1080

gattcttttg caaaaggctt caaggttgct tacaagcttg tgaaagatgg cgttttcgac   1140

aagtttatcg aggaaagata cgcaagctac aaagatggca ttggcgctga tattgtaagt   1200

ggaaaggctg acttcaagag ccttgagaag tatgcattag agcacagcca gattgtcaac   1260

aaatcaggaa gacaagagct attggagtca atcctaaatc agtatttgtt tgcagaatga   1320

<210>  21
<211>  439
<212>  PRT
<213>  Artificial sequence

<220>
<221> source
<223> /note= "Description of artificial sequence: Polypeptide sequence
            encoded by SEQ ID NO: 19"

<400>  21

Met Ala Glu Phe Phe Pro Glu Ile Pro Lys Val Gln Phe Glu Gly Lys
1               5                   10                  15

Glu Ser Thr Asn Pro Leu Ala Phe Lys Phe Tyr Asp Pro Glu Glu Val
            20                  25                  30

Ile Asp Gly Lys Pro Leu Lys Asp His Leu Lys Phe Ser Val Ala Phe
        35                  40                  45

Trp His Thr Phe Val Asn Glu Gly Arg Asp Pro Phe Gly Asp Pro Thr
    50                  55                  60

Ala Asp Arg Pro Trp Asn Arg Tyr Thr Asp Pro Met Asp Lys Ala Phe
65                  70                  75                  80

Ala Arg Val Asp Ala Leu Phe Glu Phe Cys Glu Lys Leu Asn Ile Glu
                85                  90                  95

Tyr Phe Cys Phe His Asp Arg Asp Ile Ala Pro Glu Gly Lys Thr Leu
            100                 105                 110

Arg Glu Thr Asn Lys Ile Leu Asp Lys Val Val Glu Arg Ile Lys Glu
        115                 120                 125

Arg Met Lys Asp Ser Asn Val Lys Leu Leu Leu Gly Thr Ala Asn Leu
    130                 135                 140

Phe Ser His Pro Arg Tyr Met His Gly Ala Ala Thr Thr Cys Ser Ala
145                 150                 155                 160

Asp Val Phe Ala Tyr Ala Ala Ala Gln Val Lys Lys Ala Leu Glu Ile
                165                 170                 175

Thr Lys Glu Leu Gly Gly Glu Gly Tyr Val Phe Trp Gly Gly Arg Glu
            180                 185                 190

Gly Tyr Glu Thr Leu Leu Asn Thr Asp Leu Gly Phe Glu Phe Glu Asn
        195                 200                 205

Leu Ala Arg Phe Leu Arg Met Ala Val Asp Tyr Ala Lys Lys Ile Gly
    210                 215                 220

Phe Thr Gly Gln Phe Leu Ile Glu Pro Lys Pro Lys Glu Pro Thr Lys
225                 230                 235                 240

His Gln Tyr Asp Phe Asp Val Ala Thr Ala Tyr Ala Phe Leu Lys Ser
                245                 250                 255

His Gly Leu Asp Glu Tyr Phe Lys Phe Asn Ile Glu Ala Asn His Ala
            260                 265                 270

Thr Leu Ala Gly His Thr Phe Gln His Glu Leu Arg Met Ala Arg Ile
        275                 280                 285

Leu Gly Lys Leu Gly Ser Ile Asp Ala Asn Gln Gly Asp Leu Leu Leu
    290                 295                 300

Gly Trp Asp Thr Asp Gln Phe Pro Thr Asn Val Tyr Asp Thr Thr Leu
305                 310                 315                 320

Ala Met Tyr Glu Val Ile Lys Met Gly Gly Phe Asp Lys Gly Gly Leu
                325                 330                 335

Asn Phe Asp Ala Lys Val Arg Arg Ala Ser Phe Glu Pro Glu Asp Leu
            340                 345                 350

Phe Leu Gly His Ile Ala Gly Met Asp Ser Phe Ala Lys Gly Phe Lys
        355                 360                 365

Val Ala Tyr Lys Leu Val Lys Asp Gly Val Phe Asp Lys Phe Ile Glu
    370                 375                 380

Glu Arg Tyr Ala Ser Tyr Lys Asp Gly Ile Gly Ala Asp Ile Val Ser
385                 390                 395                 400

Gly Lys Ala Asp Phe Lys Ser Leu Glu Lys Tyr Ala Leu Glu His Ser
                405                 410                 415

Gln Ile Val Asn Lys Ser Gly Arg Gln Glu Leu Leu Glu Ser Ile Leu
            420                 425                 430

Asn Gln Tyr Leu Phe Ala Glu
        435

<210>  22
<211>  439
<212>  PRT
<213>  Artificial sequence

<220>
<221> source
<223> /note= "Description of artificial sequence: Polypeptide sequence
              encoded by SEQ ID NO: 20"

<400>  22

Met Ala Glu Phe Phe Pro Glu Ile Pro Lys Val Gln Phe Glu Gly Lys
1               5                   10                  15

Glu Ser Thr Asn Pro Leu Ala Phe Lys Phe Tyr Asp Pro Glu Glu Val
            20                  25                  30

Ile Asp Gly Lys Pro Leu Lys Asp His Leu Lys Phe Ser Val Ala Phe
        35                  40                  45

Trp His Thr Phe Val Asn Glu Gly Arg Asp Pro Phe Gly Asp Pro Thr
    50                  55                  60

Ala Asp Arg Pro Trp Asn Arg Tyr Thr Asp Pro Met Asp Lys Ala Phe
65                  70                  75                  80

Ala Arg Val Asp Ala Leu Phe Glu Phe Cys Glu Lys Leu Asn Ile Glu
                85                  90                  95

Tyr Phe Cys Phe His Asp Arg Asp Ile Ala Pro Glu Gly Lys Thr Leu
            100                 105                 110

Arg Glu Thr Asn Lys Ile Leu Asp Lys Val Val Glu Arg Ile Lys Glu
        115                 120                 125

Arg Met Lys Asp Ser Asn Val Lys Leu Leu Leu Gly Thr Ala Asn Leu
    130                 135                 140

Phe Ser His Pro Arg Tyr Met His Gly Ala Ala Thr Thr Cys Ser Ala
145                 150                 155                 160

Asp Val Phe Ala Tyr Ala Ala Ala Gln Val Lys Lys Ala Leu Glu Ile
                165                 170                 175

Thr Lys Glu Leu Gly Gly Glu Gly Tyr Thr Ser Trp Gly Gly Arg Glu
            180                 185                 190

Gly Tyr Glu Thr Leu Leu Asn Thr Asp Leu Gly Phe Glu Phe Glu Asn
        195                 200                 205

Leu Ala Arg Phe Leu Arg Met Ala Val Asp Tyr Ala Lys Lys Ile Gly
    210                 215                 220

Phe Thr Gly Gln Phe Leu Ile Glu Pro Lys Pro Lys Glu Pro Thr Lys
225                 230                 235                 240

His Gln Tyr Asp Phe Asp Val Ala Thr Ala Tyr Ala Phe Leu Lys Ser
                245                 250                 255

His Gly Leu Asp Glu Tyr Phe Lys Phe Asn Ile Glu Ala Asn His Ala
            260                 265                 270

Thr Leu Ala Gly His Thr Phe Gln His Glu Leu Arg Met Ala Arg Ile
        275                 280                 285

Leu Gly Lys Leu Gly Ser Ile Asp Ala Asn Gln Gly Asp Leu Leu Leu
    290                 295                 300

Gly Trp Asp Thr Asp Gln Phe Pro Thr Asn Val Tyr Asp Thr Thr Leu
305                 310                 315                 320

Ala Met Tyr Glu Val Ile Lys Met Gly Gly Phe Asp Lys Gly Gly Leu
                325                 330                 335

Asn Phe Asp Ala Lys Val Arg Arg Ala Ser Phe Glu Pro Glu Asp Leu
            340                 345                 350

Phe Leu Gly His Ile Ala Gly Met Asp Ser Phe Ala Lys Gly Phe Lys
        355                 360                 365

Val Ala Tyr Lys Leu Val Lys Asp Gly Val Phe Asp Lys Phe Ile Glu
    370                 375                 380

Glu Arg Tyr Ala Ser Tyr Lys Asp Gly Ile Gly Ala Asp Ile Val Ser
385                 390                 395                 400

Gly Lys Ala Asp Phe Lys Ser Leu Glu Lys Tyr Ala Leu Glu His Ser
                405                 410                 415

Gln Ile Val Asn Lys Ser Gly Arg Gln Glu Leu Leu Glu Ser Ile Leu
            420                 425                 430

Asn Gln Tyr Leu Phe Ala Glu
        435

Sequence Listing

SEQ. ID. NO. 1

ATGGCTGAATTCTTTCCAGAAATCCCGAAAGTGCAGTTCGAAGGCAAAGAAA
GCACAAATCCACTTGCGTTCAAGTTCTACGATCCAGAAGAGATCATCGACGG
CAAACCCCTCAAGGACCATCTGAAGTTCTCCGTTGCCTTCTGGCACACCTTCG
TGAACGAGGGAAGGGATCCCTTCGGAGACCCAACGGCCGATCGTCCATGGA
ACAGGTACACCGATCCAATGGACAAGGCTTTTGCAAGGGTGGACGCCCTTTT
TGAATTCTGCGAAAAACTCAACATCGAGTACTTCTGCTTCCACGACAGAGAC
ATCGCTCCCGAGGGAAAAACGCTGAGGGAGACAAACAAAATTTTGGACAAA
GTAGTGGAGAGAATCAAAGAGAGAATGAAAGACAGCAACGTGAAGCTCCTC
TGGGGTACTGCAAACCTCTTTTCCCACCCAAGGTACATGCATGGTGCAGCGA
CAACCTGCAGTGCTGATGTTTTTGCGTACGCGGCCGCCCAGGTGAAAAAGGC
CCTTGAGATCACCAAAGAACTTGGAGGAGAAGGGTACACATCCTGGGGTGGA
AGAGAAGGATACGAAACACTCCTCAACACGGACCTTGGATTCGAACTTGAAA
ACCTCGCCCGCTTCCTCAGAATGGCTGTGGATTATGCAAAAAGGATCGGTTTC
ACCGGACAGTTCCTCATCGAACCAAAACCGAAAGAACCCACCAAACACCAGT
ACGACTTCGACGTTGCAACCGCCTATGCCTTCCTGAAGAGCCACGGTCTAGA
TGAGTACTTCAAGTTCAACATTGAAGCGAACCACGCGACACTTGCCGGTCAC
ACATTCCAGCACGAACTGAGGATGGCAAGGATCCTCGGAAAACTTGGAAGCA
TCGACGCCAACCAGGGAGATCTCCTGCTCGGCTGGGACACCGACCAGTTCCC
AACGAACATCTACGACACAACACTTGCCATGTACGAAGTGATCAAGATGGGG
GGATTTGACAAAGGCGGCCTTAACTTCGATGCAAAAGTAAGACGTGCTTCAT
TTGAGCCAGAAGATCTTTTCTTAGGTCACATAGCTGGAATGGATTCTTTTGCA
AAAGGCTTCAAGGTTGCTTACAAGCTTGTGAAAGATGGCGTTTTCGACAAGT
TTATCGAGGAAAGATACGCAAGCTACAAAGATGGCATTGGCGCTGATATTGT
AAGTGGAAAGGCTGACTTCAAGAGCCTTGAGAAGTATGCATTAGAGCACAGC
CAGATTGTCAACAAATCAGGAAGACAAGAGCTATTGGAGTCAATCCTAAATC
AGTATTTGTTTGCAGAATGA

SEQ. ID. NO. 2

MAEFFPEIPKVQFEGKESTNPLAFKFYDPEEIIDGKPLKDHLKFSVAFWHTFVNEG
RDPFGDPTADRPWNRYTDPMDKAFARVDALFEFCEKLNIEYFCFHDRDIAPEGK
TLRETNKILDKVVERIKERMKDSNVKLLWGTANLFSHPRYMHGAATTCSADVF
AYAAAQVKKALEITKELGGEGYTSWGGREGYETLLNTDLGFELENLARFLRMA
VDYAKRIGFTGQFLIEPKPKEPTKHQYDFDVATAYAFLKSHGLDEYFKFNIEANH
ATLAGHTFQHELRMARILGKLGSIDANQGDLLLGWDTDQFPTNIYDTTLAMYEV
IKMGGFDKGGLNFDAKVRRASFEPEDLFLGHIAGMDSFAKGFKVAYKLVKDGV
FDKFIEERYASYKDGIGADIVSGKADFKSLEKYALEHSQIVNKSGRQELLESILNQ
YLFAE

SEQ. ID. NO. 3

ATTTTGTTTAACTTTAAGAAGGAGATATACATATGAATAAATATTTTGAGAAT
GTATCTAAA

SEQ. ID. NO. 4

AGCTTGTCGACGAGCTCTTTATCATTCTGCAAACAAATACTGATT

SEQ. ID. NO. 5

ATTTTGTTTAACTTTAAGAAGGAGATATACATATGGCTGAATTCTTTCCAGAA
A

SEQ. ID. NO. 6

AGCTTGTCGACGAGCTCTTTATCACCTCAGTTCCAGAATGGTC

SEQ. ID. NO. 7

ATTTTGTTTAACTTTAAGAAGGAGATATACATATGAATAAATATTTTGAGAAC
GTATCTAAA

SEQ. ID. NO. 8

AGCTTGTCGACGAGCTCTTTATTATTCTGCAAACAAATACTGATTTAG

SEQ. ID. NO. 9

ATTTTGTTTAACTTTAAGAAGGAGATATACATATGGCAGAATTTTTCCCAGAA
A

SEQ. ID. NO. 10

AGCTTGTCGACGAGCTCTTTATCACCTCAGTTCTGCTATTGTCTTC

SEQ. ID. NO. 11

ATTTTGTTTAACTTTAAGAAGGAGATATACATATGGCTCAATCTCATTCCAGT

SEQ. ID. NO. 12

AGCTTGTCGACGAGCTCTTTATTATACTTCTAAAATGTATTGGTTCAATAT

SEQ. ID. NO. 13

ATTTTGTTTAACTTTAAGAAGGAGATATACATATGTACGAGCCCAAACCGGA

SEQ. ID. NO. 14

AGCTTGTCGACGAGCTCTTTATCACCCCCGCACCCCCAG

SEQ. ID. NO. 15

ATTTTGTTTAACTTTAAGAAGGAGATATACATATGAGCGTTCAGCCGACC

SEQ. ID. NO. 16

AGCTTGTCGACGAGCTCTTTATTAGCGGAGCCGAGCAG

SEQ. ID. NO. 17

ATTTTGTTTAACTTTAAGAAGGAGATATACATATG

SEQ. ID. NO. 18

AGCTTGTCGACGAGCTCTTTA

SEQ. ID. NO. 19

ATGGCTGAATTCTTTCCAGAAATCCCGAAAGTACAGTTCGAAGGCAAAGAAA
GCACAAATCCACTTGCGTTCAAGTTCTACGATCCAGAAGAGGTCATCGACGG
CAAACCCCTCAAGGACCATCTGAAGTTCTCCGTTGCCTTCTGGCACACCTTCG
TGAACGAGGGAAGGGATCCCTTCGGAGACCCAACGGCCGATCGTCCATGGA
ACAGGTACACCGATCCAATGGACAAGGCTTTTGCAAGGGTGGACGCCCTTTT
TGAATTCTGCGAAAAACTCAACATCGAGTACTTCTGCTTCCACGACAGAGAC
ATCGCTCCCGAGGGAAAAACGCTGAGGGAGACAAACAAAATTTTGGACAAA
GTAGTGGAGAGAATCAAAGAGAGAATGAAAGACAGCAACGTGAAGCTCCTC
TTGGGTACTGCAAACCTCTTTTCCCACCCAAGGTACATGCATGGTGCAGCGAC
AACCTGCAGTGCTGATGTTTTTGCGTACGCGGCCGCCCAGGTGAAAAAGGCC
CTTGAGATCACCAAAGAACTTGGAGGAGAAGGGTACGTATTCTGGGGTGGAA
GAGAAGGATACGAAACACTCCTCAACACGGACCTTGGATTCGAATTTGAAAA
CCTCGCCCGCTTCCTCAGAATGGCTGTGGATTATGCAAAAAGATCGGTTTCA
CCGGACAGTTCCTCATCGAACCAAAACCGAAAGAACCCACCAAACACCAGTA
CGACTTCGACGTTGCAACCGCCTATGCCTTCCTGAAGAGCCACGGTCTAGAT
GAGTACTTCAAGTTCAACATTGAAGCGAACCACGCGACACTTGCCGGTCACA
CATTCCAGCACGAACTGAGGATGGCAAGGATCCTCGGAAAACTTGGAAGCAT
CGACGCCAACCAGGGAGATCTCCTGCTCGGCTGGGACACCGACCAGTTCCCA
ACGAACGTCTACGACACAACACTTGCCATGTACGAAGTGATCAAGATGGGGG

GATTTGACAAAGGCGGCCTTAACTTCGATGCAAAAGTAAGACGTGCTTCATT
TGAGCCAGAAGATCTTTTCTTAGGTCACATAGCTGGAATGGATTCTTTTGCAA
AAGGCTTCAAGGTTGCTTACAAGCTTGTGAAAGATGGCGTTTTCGACAAGTTT
ATCGAGGAAAGATACGCAAGCTACAAAGATGGCATTGGCGCTGATATTGTAA
GTGGAAAGGCTGACTTCAAGAGCCTTGAGAAGTATGCATTAGAGCACAGCCA
GATTGTCAACAAATCAGGAAGACAAGAGCTATTGGAGTCAATCCTAAATCAG
TATTTGTTTGCAGAATGA

SEQ. ID. NO. 20

ATGGCTGAATTCTTTCCAGAAATCCCGAAAGTACAGTTCGAAGGCAAAGAAA
GCACAAATCCACTTGCGTTCAAGTTCTACGATCCAGAAGAGGTCATCGACGG
CAAACCCCTCAAGGACCATCTGAAGTTCTCCGTTGCCTTCTGGCACACCTTCG
TGAACGAGGGAAGGGATCCCTTCGGAGACCCAACGGCCGATCGTCCATGGA
ACAGGTACACCGATCCAATGGACAAGGCTTTTGCAAGGGTGGACGCCCTTTT
TGAATTCTGCGAAAAACTCAACATCGAGTACTTCTGCTTCCACGACAGAGAC
ATCGCTCCCGAGGGAAAAACGCTGAGGGAGACAAACAAAATTTTGGACAAA
GTAGTGGAGAGAATCAAAGAGAGAATGAAAGACAGCAACGTGAAGCTCCTC
TTGGGTACTGCAAACCTCTTTTCCCACCCAAGGTACATGCATGGTGCAGCGAC
AACCTGCAGTGCTGATGTTTTTGCGTACGCGGCCGCCCAGGTGAAAAAGGCC
CTTGAGATCACCAAAGAACTTGGAGGAGAAGGGTACACATCCTGGGGTGGA
AGAGAAGGATACGAAACACTCCTCAACACGGACCTTGGATTCGAATTTGAAA
ACCTCGCCCGCTTCCTCAGAATGGCTGTGGATTATGCAAAAAAGATCGGTTTC
ACCGGACAGTTCCTCATCGAACCAAAACCGAAAGAACCCACCAAACACCAGT
ACGACTTCGACGTTGCAACCGCCTATGCCTTCCTGAAGAGCCACGGTCTAGA
TGAGTACTTCAAGTTCAACATTGAAGCGAACCACGCGACACTTGCCGGTCAC
ACATTCCAGCACGAACTGAGGATGGCAAGGATCCTCGGAAAACTTGGAAGCA
TCGACGCCAACCAGGGAGATCTCCTGCTCGGCTGGGACACCGACCAGTTCCC
AACGAACGTCTACGACACAACACTTGCCATGTACGAAGTGATCAAGATGGGG
GGATTTGACAAAGGCGGCCTTAACTTCGATGCAAAAGTAAGACGTGCTTCAT
TTGAGCCAGAAGATCTTTTCTTAGGTCACATAGCTGGAATGGATTCTTTTGCA
AAAGGCTTCAAGGTTGCTTACAAGCTTGTGAAAGATGGCGTTTTCGACAAGT
TTATCGAGGAAAGATACGCAAGCTACAAAGATGGCATTGGCGCTGATATTGT
AAGTGGAAAGGCTGACTTCAAGAGCCTTGAGAAGTATGCATTAGAGCACAGC
CAGATTGTCAACAAATCAGGAAGACAAGAGCTATTGGAGTCAATCCTAAATC
AGTATTTGTTTGCAGAATGA

SEQ. ID. NO. 21

MAEFFPEIPKVQFEGKESTNPLAFKFYDPEEVIDGKPLKDHLKFSVAFWHTFVNE
GRDPFGDPTADRPWNRYTDPMDKAFARVDALFEFCEKLNIEYFCFHDRDIAPEG
KTLRETNKILDKVVERIKERMKDSNVKLLLGTANLFSHPRYMHGAATTCSADVF
AYAAAQVKKALEITKELGGEGYVFWGGREGYETLLNTDLGFEFENLARFLRMA
VDYAKKIGFTGQFLIEPKPKEPTKHQYDFDVATAYAFLKSHGLDEYFKFNIEANH
ATLAGHTFQHELRMARILGKLGSIDANQGDLLLGWDTDQFPTNVYDTTLAMYE

VIKMGGFDKGGLNFDAKVRRASFEPEDLFLGHIAGMDSFAKGFKVAYKLVKDG
VFDKFIEERYASYKDGIGADIVSGKADFKSLEKYALEHSQIVNKSGRQELLESILN
QYLFAE

SEQ. ID. NO. 22

MAEFFPEIPKVQFEGKESTNPLAFKFYDPEEVIDGKPLKDHLKFSVAFWHTFVNE
GRDPFGDPTADRPWNRYTDPMDKAFARVDALFEFCEKLNIEYFCFHDRDIAPEG
KTLRETNKILDKVVERIKERMKDSNVKLLLGTANLFSHPRYMHGAATTCSADVF
AYAAAQVKKALEITKELGGEGYTSWGGREGYETLLNTDLGFEFENLARFLRMA
VDYAKKIGFTGQFLIEPKPKEPTKHQYDFDVATAYAFLKSHGLDEYFKFNIEANH
ATLAGHTFQHELRMARILGKLGSIDANQGDLLLGWDTDQFPTNVYDTTLAMYE
VIKMGGFDKGGLNFDAKVRRASFEPEDLFLGHIAGMDSFAKGFKVAYKLVKDG
VFDKFIEERYASYKDGIGADIVSGKADFKSLEKYALEHSQIVNKSGRQELLESILN
QYLFAE

## Claims

1. An isolated polypeptide **characterised in that** it comprises an amino acid sequence having at least 80% identity to a sequence selected from the group consisting of SEQ ID NO 2, SEQ ID NO 21 and SEQ ID NO 22 over the entire length of said sequence, fragments and variants thereof.

2. An isolated polypeptide comprising an amino acid sequence selected from the group consisting of SEQ ID NO 2, SEQ ID NO 21 and SEQ ID NO 22, fragments and variants thereof.

3. An isolated polypeptide selected from the group consisting of SEQ ID NO 2, SEQ ID NO 21 and SEQ ID NO 22, fragments and variants thereof.

4. The polypeptide according to any one of claims 1-3, **characterised in that** it is capable of converting glucose to fructose.

5. An isolated polynucleotide, **characterised in that** it encodes the polypeptide of any one of claims 1-4.

6. An isolated polynucleotide, **characterised in that** it has at least 80% identity to the polynucleotide of claim 5, over the entire length of said sequence.

7. An isolated polynucleotide, **characterised in that** it comprises the polynucleotide of claim 5 or claim 6.

8. An isolated polynucleotide **characterised in that** it comprises a nucleotide sequence having at least 80% identity to a sequence selected from the group consisting of SEQ ID NO 1, SEQ ID NO 19 and SEQ ID NO 20, over the entire length of said sequence, fragments and variants thereof.

9. An isolated polynucleotide comprising a nucleotide sequence selected from the group consisting of SEQ ID NO 1, SEQ ID NO 19 and SEQ ID NO 20, fragments and variants thereof.

10. An isolated polynucleotide selected from the group consisting of SEQ ID NO 1, SEQ ID NO 19 and SEQ ID NO 20, fragments and variants thereof.

11. An isolated polynucleotide **characterised in that** it is complementary to the polynucleotide of any one of claims 5-10.

12. A isolated polypeptide encoded by the polynucleotide of any one of claims 5-11.

13. An expression vector **characterised in that** it comprises a polynucleotide according to any one of claims 5-11.

14. A recombinant host cell **characterised in that** it comprises the vector of claims 13.

15. A process for producing a polypeptide according to any one of claims 1-4 or 12, comprising the steps of culturing a host cell of claim 14 under conditions sufficient for the production of said polypeptide; and recovering the polypeptide from the culture medium.

16. A polypeptide obtainable according to the process of claim 15.

17. An antibody that selectively binds the polypeptide of any one of claims 1 to 4, 12 or 16.

18. A method for producing a fructose syrup, comprising the step of contacting a glucose-containing composition with a polypeptide according to any one of claims 1 to 4, 12 or 16 under conditions effective to convert glucose to fructose.

19. The method according to claim 18, **characterised in that** the reaction temperature is in the range of from 60 to 110°C, preferably 80 to 100°C.

20. The method according to claim 18 or claim 19, **characterised in that** the pH of the reaction mixture is in the range of from 4.5 to 8, preferably 5 to 7, more preferably 5 to 6.

21. The method according to any one of claims 18-20, **characterised in that** the reaction is carried out in the presence of at least one bivalent cation selected from the group consisting of Mg²⁺ and Mn²⁺.

22. A fructose syrup obtainable according to the method of any one of claims 18-21, preferably a high-fructose syrup, even more preferably, a high fructose corn syrup.
